# EUROPEAN PATENT APPLICATION

(11) **EP 4 257 148 A1**
(43) Date of publication of application: **11.10.2023**
(21) Application number: 21902942.8
(22) Date of filing: 01.09.2021
(51) Int. Cl.: A61K 45/00, A61K 9/08, A61K 31/192, A61K 31/575, A61P 27/02, A61P 27/10, C12Q 1/02, C12Q 1/68, G01N 33/15, G01N 33/50

(54) **EYEDROPS FOR TREATING SCLERAL THINNING AND SCREENING METHOD FOR THERAPEUTIC AGENT OF SCLERAL THINNING**

(30) Priority: 11.12.2020 JP 2020205490
(71) Applicant: Tsubota Laboratory, Inc., Tokyo 160-0016 (JP)
(72) Inventor: TSUBOTA Kazuo, Tokyo 160-0016 (JP); KURIHARA Toshihide, Tokyo 160-0016 (JP); IKEDA Shinichi, Tokyo 160-0016 (JP); MORI Kiwako, Tokyo 160-0016 (JP); JIANG Xiaoyan, Tokyo 160-0016 (JP)
(74) Representative: Mewburn Ellis LLP
(86) International application number: PCT/JP2021/032068
(87) International publication number: WO 2022/123837

(57) **Abstract**

The present invention provides a screening method for searching a component inhibiting or treating scleral thinning, and eyedrops that contain the active ingredient, and hence can inhibit excessive thinning of the sclera, and as a result, can treat a posterior segment eye disease associated with the scleral thinning. The problems are solved by eyedrops containing, as an active ingredient, a component capable of simultaneously inhibiting the PERK pathway and/or the ATF6 pathway. The problems are solved by a screening method for a component capable of simultaneously inhibiting the PERK pathway and/or the ATF6 pathway, including a step of contacting a candidate substance with an eye-derived cell, and a step of selecting the candidate substance using, as an indicator, influence on scleral thinning in the cell.

## Description

### Technical Field

The present invention relates to eyedrops for treating scleral thinning, in an adult eye in which myopia has progressed, comprising an active ingredient capable of normally maintaining the shape of the eyeball by inhibiting scleral thinning affecting the shape of the eyeball, and capable of treating an eye disease associated with scleral thinning, and a screening method for the therapeutic agent of scleral thinning.

### Background Art

According to recent research on myopia and high myopia, the number of people with myopia is expected to remarkably increase worldwide, and the number of people with myopia is expected to be about five billion, and the number of people with high myopia is expected to be over about ten billion in 2050 (see Non Patent Literature 1).

Besides, as a result of some epidemiological studies in Japan, it has been found that the prevalence of -6 D or higher myopia is about 5%, and according to Tajimi Study, that is, the epidemiological study carried on the general public, myopic macular degeneration caused by high myopia is the third cause of blindness (see Non Patent Literature 2).

It is known that the sclera playing a crucial role in maintaining the shape of the eyeball is thinned by such high myopia, and the scleral thinning is known to cause various eye diseases (see Non Patent Literature 2). These eye diseases associated with scleral thinning are classified into posterior segment eye diseases such as myopic macular degeneration, cataract, and eye movement disorder. In all the eye diseases associated with scleral thinning, it is not easy to treat or reproduce optic nerves and the lens once damaged, and hence, the radical treatment is inhibition of scleral thinning (deformation of the eyeball) caused by excessive axial elongation, that is, root cause of these diseases.

For example, for myopic choroidal neovascularization (myopic CNV), that is, one of the eye diseases associated with scleral thinning, aflibercept and ranibizumab are known as existing drugs for inhibiting neovascularization. These antibody preparations are, however, capable of inhibiting neovascularization, but have no effect of treating scleral thinning, and are known to have bad prognosis such as cicatrization of the choroid. Accordingly, fundamental treatment at an early stage before such diseases become serious (reduction of mechanical pressure to the posterior segment of the eye) is being strongly demanded.

### Citation List

### Patent Literature

Patent Literature 1: International Publication No. WO2018/164113

### Non Patent Literature

Non Patent Literature 1: Global prevalence of myopia and high myopia and temporal trends from 2000 through 2050, Ophthalmology, Vol. 123, Number 5, May 2016
Non Patent Literature 2: Zoka suru Kinshi/Kyodo Kinshi (Increasing Myopia/High Myopia), Journal of Clinical and Experimental Medicine, Vol. 253, Issue 2, 159-161, 2015
Non Patent Literature 3: Takashi Fujikado, "Nihon Ganka Gakkai Senmon-i Seido Shogai Kyouiku Koza (Japanese Ophthalmological Society Board Certification System Lifelong Education Course) Review 54, Shoni no Kinshi no Shinko Boshi (Prevention of Progression of Child Myopia), Japanese Journal of Ophthalmology, vol. 117, No. 4, pp. 397-406 (April 10, 2013)
Non Patent Literature 4: Posterior Staphyloma in Pathologic Myopia, Progress in Retinal and Eye Research, 70 (2019) 99-100
Non Patent Literature 5: Pathogenesis and Prevention of Worsening Axial Elongation in Pathological Myopia, Clinical Ophthalmology 2020: 14 853-873
Non Patent Literature 6: Jiang, X., et.al., A highly efficient murine model of experimental myopia, Scientific reports 8, 2026, doi: 10.1038/s 41598-018-20272-w (2018)
Non Patent Literature 7: Mori, K., et.al., Oral crocetin administration suppressed refractive shift and axial elongation in a murine model of lens-induced myopia, Scientific reports 9, 295, doi: 10.1038/s41598-018-36576-w (2019)

### Summary of Invention

### Technical Problem

In recent years, factors for scleral thinning (herein also referred to as deformation of the eyeball) have become clear. As a result of earnest studies on the group of these factors by the present inventors, it has been found that UPR (unfolded protein response) gene group is involved in axial elongation (see Patent Literature 1). As the group of these factors, three factors of PERK (PKR-like endoplasmic reticulum kinase), ATF6 (activating transcription factor 6), and IRE1 (inositol requiring 1) are known, but it has been unknown how these factors are to be controlled to inhibit or treat scleral thinning. In other words, it has been regarded that it is very crucial and difficult for therapeutic strategies how the gene group is to be controlled.

### Solution to Problem

The present inventors examined a test system for simulating adult scleral thinning (excessive axial elongation) for studying a mechanism of scleral thinning caused after completing myopia induction in a mouse, and a component effective for treating the scleral thinning. It is strongly expected that such a component can inhibit scleral thinning, and additionally can treat an eye disease associated with scleral thinning.

An object of the present invention is to provide a screening method for searching a component capable of inhibiting scleral thinning caused by adult high myopia, and to provide eyedrops capable of treating scleral thinning and an eye disease associated therewith by using an active ingredient obtained by the screening method.

Specifically, the present invention provides the following:
[1] Eyedrops for treating scleral thinning, comprising an inhibitor of PERK (PKR-like endoplasmic reticulum kinase) pathway and/or ATF6 (activating transcription factor 6) pathway as an active ingredient.
[2] The eyedrops according to [1] described above, wherein the inhibitor is at least one selected from the group consisting of phenylbutyric acid and pharmacologically acceptable salts thereof.
[3] The eyedrops according to [1] or [2] described above, wherein the inhibitor is sodium phenylbutyrate.
[4] The eyedrops according to any one of [1] to [3] described above, wherein a content of the inhibitor is 0.01 to 5% by mass based on a total amount of the eyedrops.
[5] The eyedrops according to any one of [1] to [4] described above, wherein the treatment of scleral thinning is treatment of a posterior segment eye disease caused by the scleral thinning.
[6] The eyedrops according to [5] described above, wherein the posterior segment eye disease is myopic macular degeneration, myopic chorioretinal atrophy, myopic choroidal neovascularization, or myopic optic neuropathy.
[7] A screening method for a therapeutic agent of scleral thinning, comprising a step of contacting a candidate substance with an eye-derived cell; and a step of selecting the candidate substance by using, as an indicator, change in a protein and/or a gene of a signal transduction system of PERK and/or ATF6 in the cell.

### Advantageous Effects of Invention

According to the present invention, a screening method for searching a component capable of inhibiting scleral thinning can be provided. Besides, by using an active ingredient obtained by the screening method, eyedrops capable of treating scleral thinning and an eye disease associated therewith can be provided.

### Brief Description of Drawings

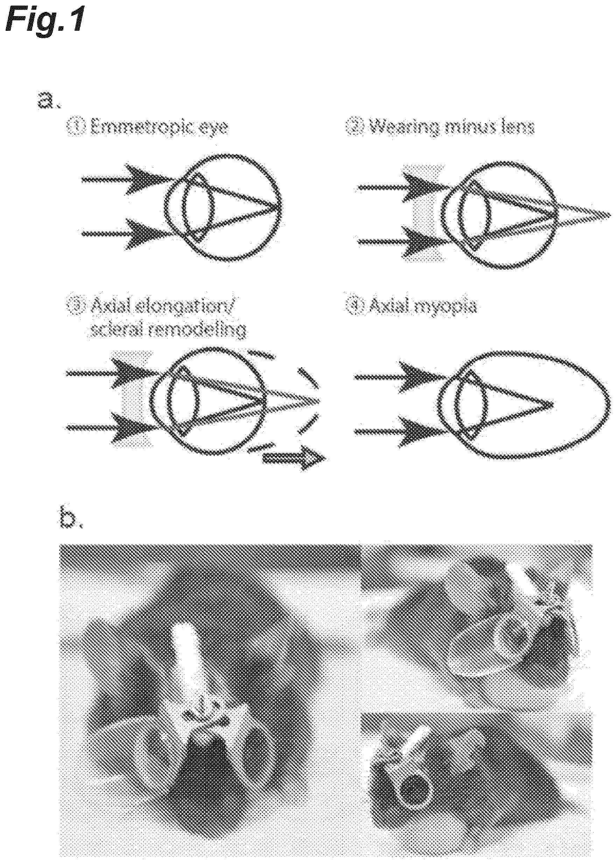

[Figure 1] Figure 1 is an explanatory diagram of myopia induction in a mouse, wherein Figure 1(a) illustrates schematical structural views of the myopia induction, and Figure 1(b) illustrates photographs of myopia induced mice.

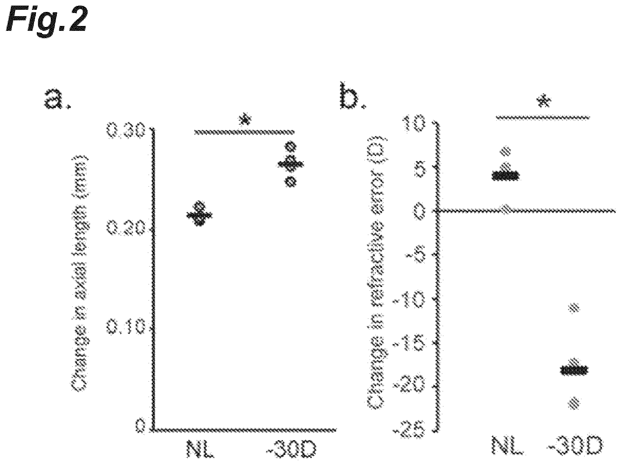

[Figure 2] Figure 2 is a graph illustrating that myopia induction induces axial elongation and refraction change in the sclera, wherein Figure 2(a) illustrates change of axial elongation obtained by myopia induction for 3 weeks in mice (n = 4) (*p < 0.05), and Figure 2(b) illustrates refraction change obtained by myopia induction for 3 weeks in mice (n = 4) (*p < 0.05).

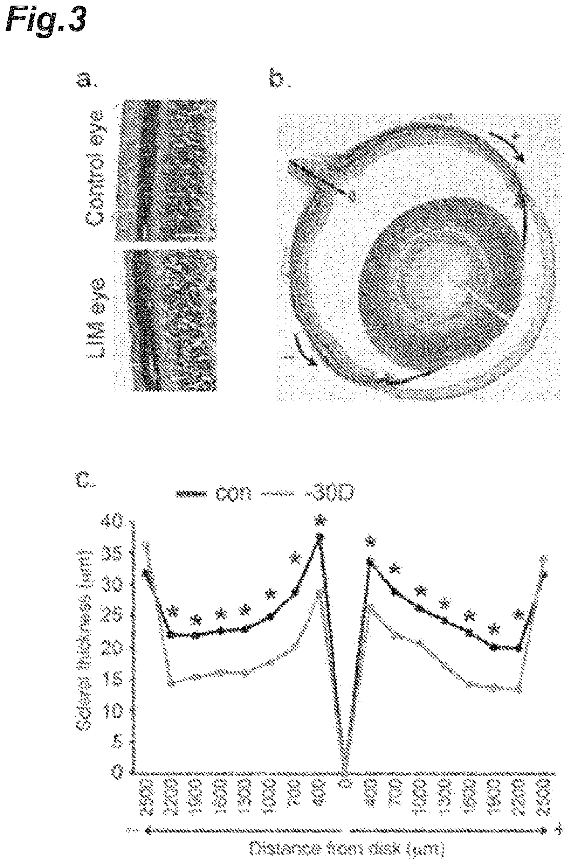

[Figure 3] Figure 3 is an explanatory diagram of ophthalmological and cytological changes obtained after myopia induction. Figure 3(a) illustrates hematoxylin and eosin staining of a control eye and a myopia-induced eye, wherein a yellow bar indicates the thickness of the sclera (n = 5 in each group, scale bar = 50 µm). Figure 3(b) is an explanatory diagram of measurement of a sclera thickness, and with the position of the optic disc set as "0", the sclera thickness was measured in positions away from the optical disc above (+) and below (-) by distances (of 400 µm , 700 µm, 1000 µm, 1300 µm, 1600 µm, 1900 µm , 2200 µm, and 2500 µm). Figure 3(c) is a graph of the measurement results of the sclera thickness.

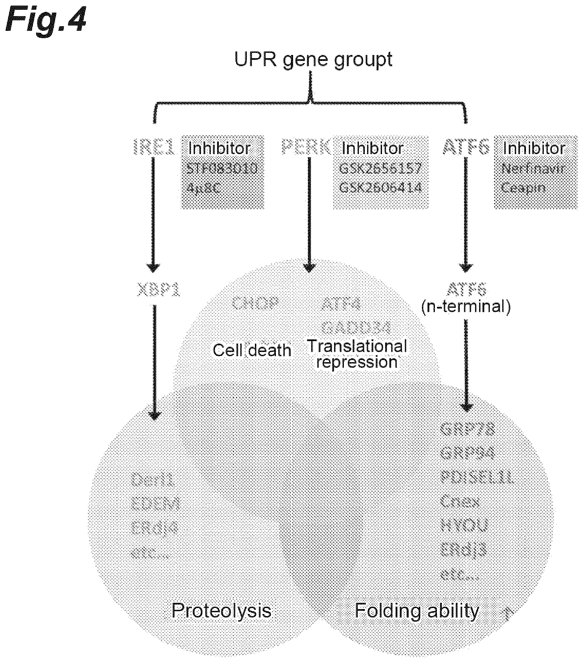

[Figure 4] Figure 4 is an explanatory diagram illustrating various inhibitors for the PERK pathway, the ATF6 pathway, and IRE1 pathway corresponding to UPR genes.

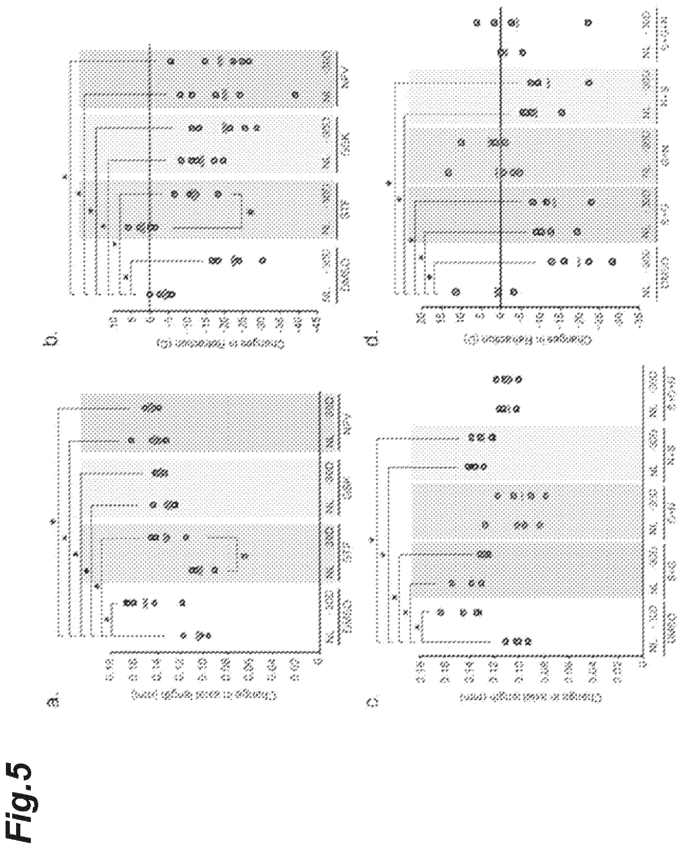

[Figure 5] Figure 5 illustrates graphs indicating axial elongation and refraction change (myopia) caused in mice by eyedrop of various inhibitors for the PERK pathway, the ATF6 pathway and the IRE1 pathway. Figure 5(a) is a graph illustrating influence on the axial elongation caused by eyedrop administration of single one of STF080310 (STF), GSK2656157 (GSK) and nelfinavir (NEV) (n = 5 in each group), and illustrating results of comparison with a DMSO eye-dropped NL (= no lens) group (*p < 0.05), and results of comparison with a STF eye-dropped NL group or a -30 D lens wearing group (#p < 0.05). Figure 5(b) illustrates results of influence on myopic refraction caused by eyedrop administration of single one of STF, GSK and NFV (n = 5 in each group), and illustrates results of comparison with a DMSO eye-dropped NL group (*p < 0.05), and results of comparison with a STF eye-dropped NL group or a -30 D lens wearing group (#p < 0.05). Figure 5(c) illustrates results of influence on the axial elongation caused by eyedrop administration of STF, GSK and NFV in combination (n = 4 in each group), and illustrates results of comparison with a DMSO eye-dropped NL group (*p < 0.05). Figure 5(d) illustrates results of influence on myopic refraction caused by eyedrop administration of STF, GSK and NFV in combination (n = 4 in each group), and illustrates results of comparison with a DMSO eye-dropped NL group (*p < 0.05).

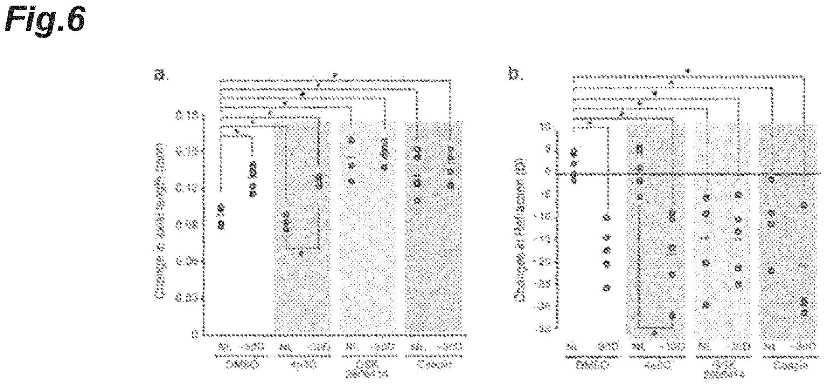

[Figure 6] Figure 6 illustrates graphs indicating axial elongation and refraction change (myopia) caused in mice by eyedrop administration of various inhibitors, different from those of Figure 5, for the PERK pathway, the ATF6 pathway and the IRE1 pathway. Figure 6(a) illustrates graphs of influence on axial elongation caused by eyedrop administration of single one of 4µ8C, GSK2606414, and Ceapin-A7 (n = 5 in each group), and illustrates results of comparison with a DMSO eye-dropped NL (= no lens) group (*p < 0.05), and results of comparison with a 4µ8C eye-dropped NL group or a -30 D lens wearing group (#p < 0.05). Figure 6(b) illustrates influence on myopic refraction caused by eyedrop administration of single one of 4µ8C, GSK2606414, and Ceapin-A7 (n = 5 in each group), and illustrates results of comparison with a DMSO eye-dropped NL (= no lens) group (*p < 0.05), and results of comparison with a 4µ8C eye-dropped NL group or a -30 D lens wearing group (#p < 0.05).

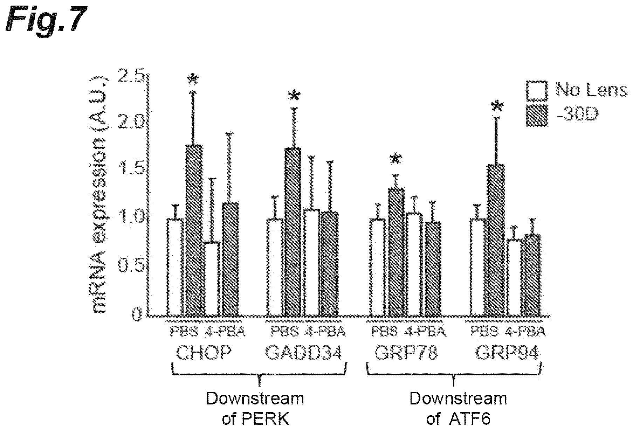

[Figure 7] Figure 7 illustrates a graph indicating that myopia induction in a mouse induces increase of UPR gene expression in the sclera, wherein UPR gene expression is measured by quantitative PCR in the sclera (n = 6 in each group) having PBS intraperitoneally injected (PBS) or having sodium phenylbutyrate administered (4-PBA; 200 mg/kg/day), for showing results that the gene expression is increased in myopia induced eyes (gray column) as compared with control eyes (white column), and that the increase is inhibited by 4-PBA (*p < 0.05).

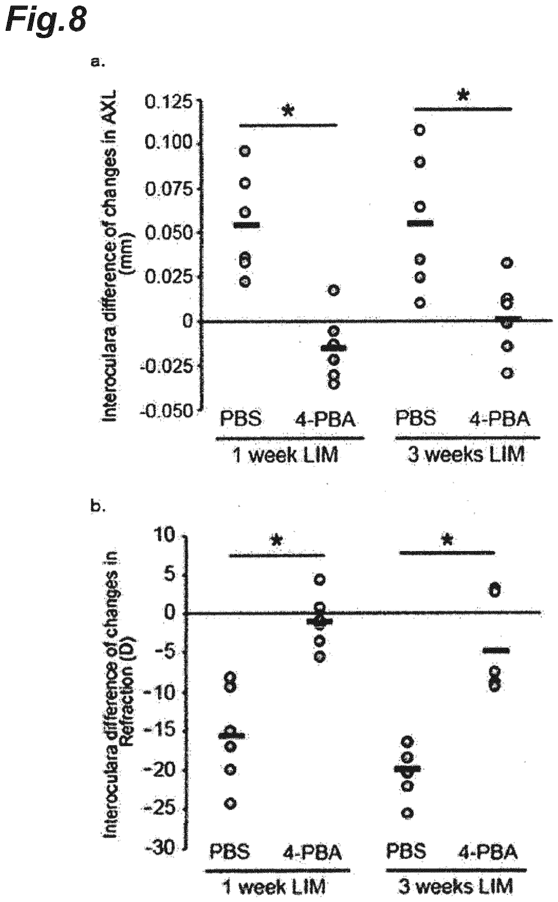

[Figure 8] Figure 8 illustrates graphs indicating that myopia induction in mice induces axial elongation and myopic refraction, Figure 8(a) illustrates results indicating that the axial elongation is inhibited by intraperitoneal injection of phenylbutyric acid in 1st week and 3rd week of the myopia induction (LIM) (4-PBA; 200 mg/kg/day) (n = 6 in each group, *p < 0.05), and Figure 8(b) illustrates results indicating that the myopic refraction is inhibited by 4-PBA administration in 1st week and 3rd week of the myopia induction (LIM) (n = 6 in each group, *p < 0.05).

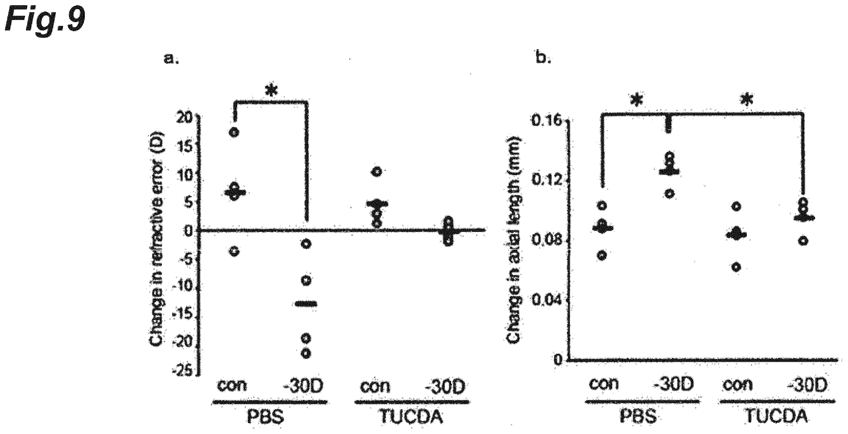

[Figure 9] Figure 9 illustrates graphs indicating that myopia induction in mice induces axial elongation and myopic refraction, wherein Figure 9(a) is a graph illustrating that the myopic refraction is inhibited by intraperitoneal injection of tauroursodeoxycholic acid (TUDCA; 100 mg/kg) (n = 4 in each group, *p < 0.05), and Figure 9(b) illustrates that the axial elongation is inhibited by TUDCA (n = 4 in each group, *p < 0.05).

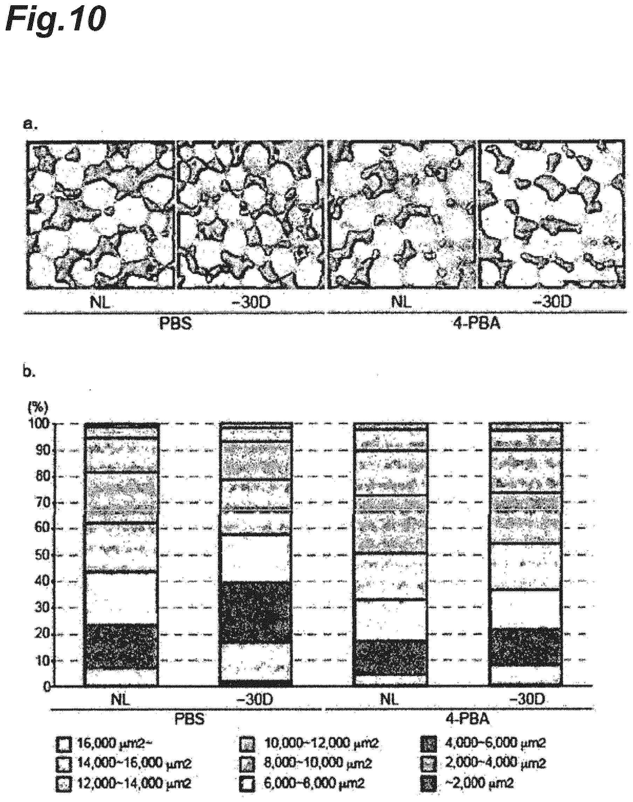

[Figure 10] Figure 10(a) illustrates transmission electron microscope images of scleral collagen fibers (each being a representative image of biologically independent three samples) obtained by eyedrop administration of PBS or 4-PBA to C57BL6J mice wearing no lens (NL) or wearing a -30 D lens. Figure 10(b) illustrates results of scleral collagen fiber areas (n = biologically independent three samples). The fiber area is measured based on five images obtained from one sclera with Image J software.

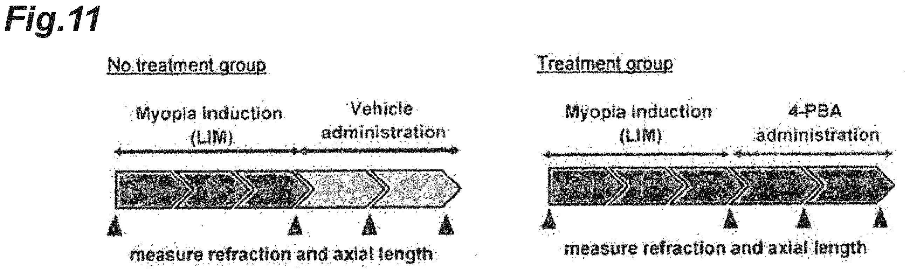

[Figure 11] Figure 11 is an explanatory diagram illustrating that eye-dropped 4-PBA is effective in myopia in an adult in which myopia progression has been completed, and is a schematic diagram illustrating an experimental scheme of eyedrop administration after completing a 3-week myopia induction (LIM) period (simulating a myopia period in an adult).

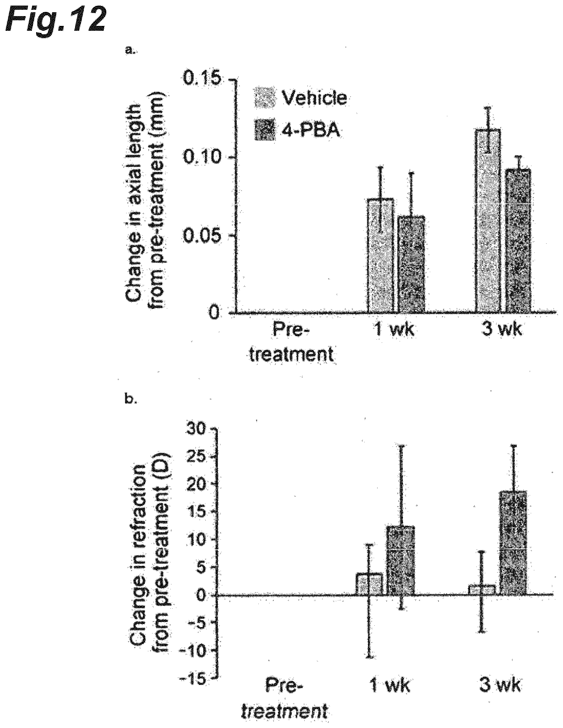

[Figure 12] Figure 12 illustrates myopia inhibition effect obtained by eyedrop administration of 4-PBA after completing a myopia induction period of a mouse corresponding to a myopia period in an adult, wherein Figure 12 (a) illustrates results of axial elongation obtained in 1st week (1wk) or 3rd week (3wk) after pre-treatment in vehicle (n = 7) and 4-PBA (n = 5) eye-dropped groups, and Figure 12(b) illustrates refraction change (n = 5) obtained by similar eyedrop administration.

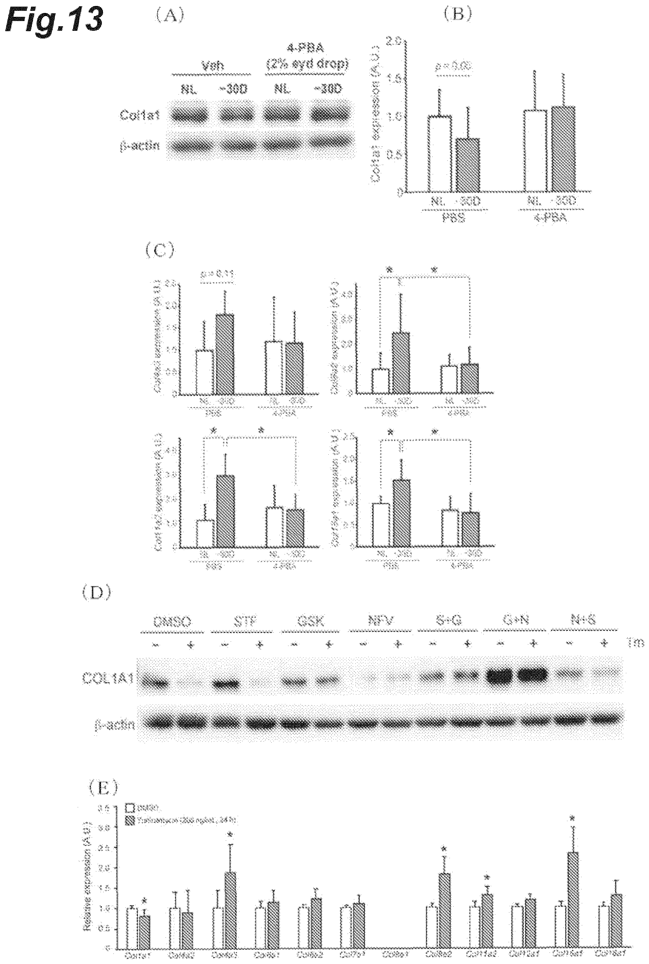

[Figure 13] Figure 13 illustrates results, obtained in Test Example 7, of influence of 4-PBA eyedrop administration or UPR gene inhibitor administration on reduction of major collagen components.

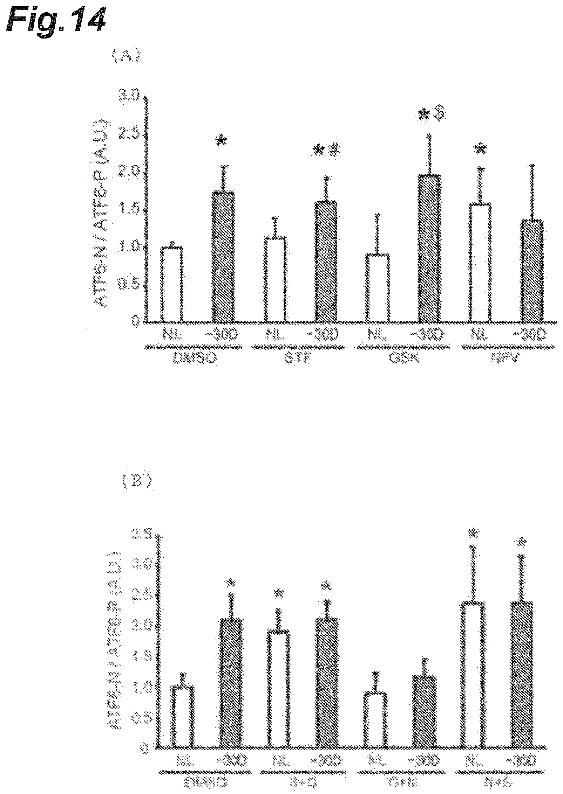

[Figure 14] Figure 14 illustrates graphs, obtained in Test Example 8, of evaluation of involvement of the ATF6 pathway in treatment of scleral thinning and a posterior segment eye disease associated therewith.

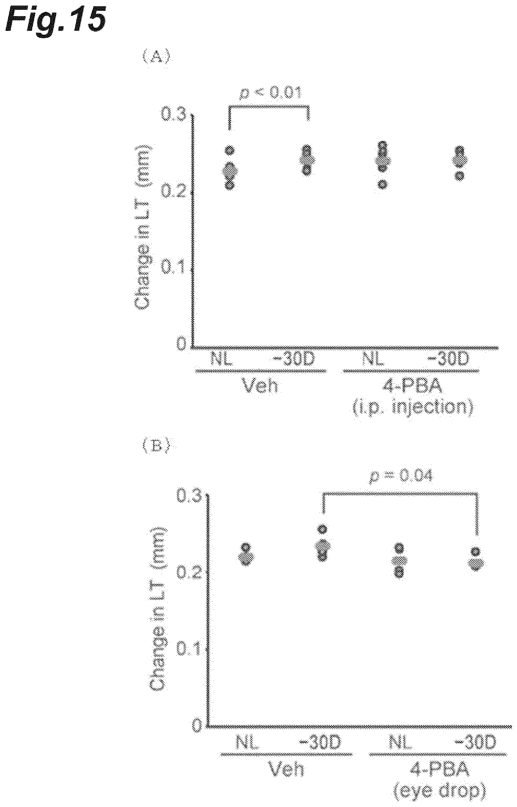

[Figure 15] Figure 15 illustrates graphs, obtained in Test Example 9, of influence of myopia induction on lens thickening depending on a difference in dosage form.

### Description of Embodiments

The present invention will now be described in detail. The present invention is not limited to the following embodiments and experimental examples but encompasses various modification examples and application examples within the scope of the present invention.

### [Eyedrops for Treating Scleral Thinning]

Eyedrops for treating scleral thinning and an eye disease associated therewith of the present invention contains, as an active ingredient, an inhibitor of the PERK (PKR-like endoplasmic reticulum kinase) pathway and the ATF6 (activating transcription factor 6) pathway.

### (Therapeutic Agent of Scleral Thinning)

As described above, excessive axial elongation causes scleral thinning (deformation of the eyeball), and is involved in onset and/or exacerbation of scleral thinning and an eye disease associated therewith. As described in experimental examples below, it has been confirmed that scleral thinning that can be a cause of deformation of the eyeball is caused in a mouse myopia induction model, and it has been suggested that the mechanism is expression increase of specific genes of UPR gene group, and as a result, collagen fibers narrowing is caused in the sclera to cause scleral thinning. Therefore, a substance having an effect of inhibiting scleral thinning can be an active ingredient.

In other words, compounds, and nucleic acids such as antisense oligonucleotide and siRNA that target and inhibit genes and/or proteins involved in scleral thinning can be blended in eyedrops as a component effective for treatment of scleral thinning and an eye disease associated therewith.

Herein, the inhibitor of the PERK pathway and the ATF6 pathway refers to a substance having an inhibitory effect on both the signal transduction system of PERK (the PERK pathway) and the signal transduction system of ATF6 (the ATF6 pathway). The effect of inhibiting these signal transduction systems can be evaluated, as described in Examples below, by a known method using, as an indicator, change in a gene and/or a protein involved in these signal transduction systems.

### (Inhibitor of PERK Pathway and/or ATF6 Pathway)

As described above, as a factor for scleral thinning (pathologic axial elongation), a gene pathway responding to an unfolded protein that is an abnormal protein in the endoplasmic reticulum is involved in the pathologic axial elongation. As the gene pathway, three pathways of the PERK pathway, the ATF6 pathway, and the IRE1 pathway are known, and as described in experimental examples below, it has been newly found that it is essential for myopia inhibition to inhibit at least the ATF6 pathway. It has been also newly found that an effect of inhibiting myopia progression is further increased by inhibiting the PERK pathway and the ATF6 pathway among these three pathways. It has been also confirmed that when only one of the PERK pathway and the ATF6 pathway is inhibited, the other pathway may be activated in compensation. Therefore, in one embodiment, although not limited, a substance having an inhibitory effect on both the PERK pathway and the ATF6 pathway can be an active ingredient for inhibiting pathologic axial elongation (scleral thinning).

In other words, a compound that targets and reduces a gene or a protein involved in signal transduction of PERK and/or ATF6, or a nucleic acid such as antisense oligonucleotide or siRNA that reduces protein expression in the PERK pathway and/or the ATF6 pathway can be blended in eyedrops as an ingredient effective for treating scleral thinning.

Herein, the inhibitor of the PERK pathway or the ATF6 pathway refers to a substance having an inhibitory effect on the signal transduction system of PERK or the signal transduction system of ATF6 in the endoplasmic reticulum. The inhibitory effect on these signal transduction systems can be evaluated by using, as an indicator, change in a gene and/or a protein involved in these signal transduction systems by a method described in experimental examples below, or by a known method.

In evaluation of expression of a gene or expression of a protein of a factor involved in the signal transduction system of PERK, the evaluation can be performed in accordance with that the expression of the factor is varied by at least 1% by a candidate substance as compared with a control not having the candidate substance added thereto.

Besides, in evaluation of expression of a gene or expression of a protein of a factor involved in the signal transduction system of ATF6, the evaluation can be performed in accordance with that the expression of the factor is varied by at least 1% by a candidate substance as compared with a control not having the candidate substance added thereto.

PERK is endoplasmic reticulum transmembrane kinase, and examples of the factor involved in the signal transduction include eIF2a (eukaryotic initiation factor 2α), ATF4 (activating transcription factor 4), CHOP (C/EBP homologous protein), and GADD34 (growth arrest DNA and damage protein 34).

Besides, ATF6 is a membrane-bound transcription factor belonging to the CREB/ATF family, and examples of the factor involved in the signal transduction include BiP (binding immunoglobulin protein, also referred to as "GRP78"), Txndc12 (thioredoxin domain containing 12, also referred to as "ERp18"), S1P (site-1 protease), and S2P (site-2 protease).

In experimental examples described below, at least one selected from the group consisting of phenylbutyric acid, tauroursodeoxycholic acid, and pharmacologically acceptable salts thereof has been found by screening a component capable of inhibiting both the PERK pathway and the ATF6 pathway. The component is, however, not limited to this, but a component newly specified as a component inhibiting at least the ATF6 pathway, and a component newly specified as a component inhibiting the PERK pathway and the ATF6 pathway can be used. The inhibitor of the PERK pathway and the ATF6 pathway is not limited, and from the viewpoint of solubility in eyedrops, sodium phenylbutyrate is preferred. As described in experimental examples below, sodium phenylbutyrate is preferred because not only the ATF6 pathway but also the PERK pathway can be inhibited. The inhibitor of the PERK pathway and/or the ATF6 pathway may be synthesized by a known method to be used, or a commercially available product may be obtained to be used.

Herein, the "pharmaceutically acceptable salt" is not especially limited, and specific examples include organic acid salts, inorganic acid salts, organic base salts, and inorganic base salts. Examples of the organic acid salts include monocarboxylic acid salts such as acetic acid salt, trifluoroacetic acid salt, butyric acid salt, palmitic acid salt, and stearic acid salt; polycarboxylic acid salts such as fumaric acid salt, maleic acid salt, succinic acid salt, and malonic acid salt; oxycarboxylic acid salts such as lactic acid salt, tartaric acid salt, and citric acid salt; and organic sulfonic acid salts such as methanesulfonic acid salt, toluenesulfonic acid salt, and tosic acid salt. Examples of the inorganic acid salts include hydrochloric acid salt, sulfuric acid salt, nitric acid salt, hydrobromic acid salt, and phosphoric acid salt. Examples of a salt with an organic base include salts with organic amines such as methyl amine, triethyl amine, triethanol amine, diethanol amine, morpholine, piperazine, pyrrolidine, tripyridine, picoline, and ethylene diamine. Examples of a salt with an inorganic base include various salts such as ammonium salt; and salts with alkali metals such as sodium and potassium, alkaline earth metals such as calcium and magnesium, and metals such as aluminum. One of these salts may be singly used, or two or more of these may be used in an optional combination. The "pharmaceutically acceptable salt" may include a solvate or a hydrate of a salt.

A content of the inhibitor of the PERK pathway and/or the ATF6 pathway can be appropriately changed depending on an administration method, a dosage, the type of additive and the like. For example, the content is preferably 0.01% by mass or more, more preferably 0.05% by mass or more, further preferably 0.1% by mass or more, and particularly preferably 0.2% by mass or more based on the total amount of the eyedrops. Besides, the content of the inhibitor of the PERK pathway and/or the ATF6 pathway is, for example, preferably 5% by mass or less, more preferably 4% by mass or less, further preferably 3% by mass or less, and particularly preferably 2% by mass or less based on the total amount of the eyedrops. Besides, the content of the inhibitor of the PERK pathway and/or the ATF6 pathway is, for example, preferably 0.01 to 5% by mass, more preferably 0.05 to 4% by mass, further preferably 0.1 to 3% by mass, and particularly preferably 0.2 to 2% by mass based on the total amount of the eyedrops.

When at least one selected from the group consisting of phenylbutyric acid and pharmacologically acceptable salts thereof is used as the therapeutic agent of scleral thinning, the content is, for example, preferably 0.01 to 5% by mass, more preferably 0.05 to 4% by mass, further preferably 0.1 to 3% by mass, and particularly preferably 0.2 to 2% by mass based on the total amount of the eyedrops.

### [Usage]

The axial length rapidly elongates after birth up to about 2 years old, and thereafter gradually elongates. Such axial length elongation along with growth is designated as "physiological axial elongation", and is an indispensable phenomenon for development of the eye. Continuous elongation of the axial length even after school age or older leads, however, to progression of myopia, and hence is regarded as "pathologic axial elongation". For example, in pathologic axial elongation, elongation of the axial length by 1 mm in an adult eye leads to increase of the degree of myopia by about 3.0 D.

The eyedrops of the present invention are used for treatment of scleral thinning and an eye disease associated therewith. Herein, an eye disease associated with scleral thinning refers to a disease caused by organic excessive elongation of the axial length owing to high myopia. In high myopia, thinning of the sclera that keeps the shape of the eyeball is said to be an early symptom of the associated eye disease (see Non Patent Literature 4).

In examples described below, even during 3 weeks after completing myopia induction in a myopia-induced mouse, the eye axis continuously elongated, and scleral thinning was caused. In other words, the pathologic axial elongation and scleral thinning caused after completing the myopia induction was confirmed to continue even in a period corresponding to adulthood following the myopia progression in a child, which was suggested to be the mechanism of the onset of scleral thinning and an eye disease associated therewith. Therefore, it is presumed that when scleral thinning is evaluated in accordance with the thickness of the sclera, the thickness of collagen fibers in the sclera, or expression or the like in the sclera of a collagen-related gene/protein (at least one selected from the group consisting of COL1A1, COL4A3, COL8A2, COL11A2, and COL15A1) to screen a component capable of inhibiting the thinning, the resultant can be utilized for inhibiting deformation of the eyeball and for treating scleral thinning and an eye disease associated therewith.

In examples described below, the PERK pathway and the ATF6 pathway were overactive after completing the myopia induction in a myopia induced mouse, and axial elongation corresponding to the cause of scleral thinning was caused. In other words, it was confirmed that the pathologic axial elongation and scleral thinning caused by the myopia induction continues in a period corresponding to adulthood following the myopia progression in a child, which was suggested to be the mechanism of the onset of scleral thinning and an eye disease associated therewith. Therefore, it is presumed that when scleral thinning is evaluated in accordance with simultaneous overactivity of the PERK pathway and the ATF6 pathway to screen a component capable of inhibiting these, the resultant can be utilized for inhibiting deformation of the eyeball and for treating scleral thinning and an eye disease associated therewith.

Examples of the eye disease associated with scleral thinning include eye diseases such as myopic macular degeneration, myopic chorioretinal atrophy, myopic choroidal neovascularization, myopic optic neuropathy, myopic retinopathy, chorioretinal atrophy, macular hemorrhage, myopic traction maculopathy, myopic maculopathy, myopic macular lesion, myopic macular separation, myopic refractive scotoma, myopic conus, myopic foveoschisis, diffuse atrophic lesion, focal atrophic lesion, Lacquer cracks, posterior staphyloma, retinal detachment, macular hole, tilted disc syndrome, myopic astigmatism, fixed esotropia, mechanical abduction restriction, esotropia, and high myotic strabismus.

Among these eye diseases associated with scleral thinning, the disease is preferably a posterior segment eye disease from the viewpoint of its strong causal relationship with scleral thinning (deformation of the eyeball). Besides, from the viewpoint that there is a definite and direct causal relationship therebetween, the present invention is applied preferably to myopic macular degeneration, myopic chorioretinal atrophy, myopic choroidal neovascularization, or myopic optic neuropathy.

### (Dosage Form)

A composition of the present invention is used in the form of eyedrops. In the present invention, the dosage form of the eyedrops for treating scleral thinning is not limited, and examples include aqueous eyedrops, eyedrops dissolved before use, suspension eyedrops, oily eyedrops, and an eye ointment. Among these, the dosage form is preferably aqueous eyedrops from the viewpoint of remarkably exhibiting the effects of the present invention.

In the eyedrops, other active ingredients (such as a pharmacologically active ingredient, and a physiological active ingredient) can be blended in addition to the above-described component. The type of such an ingredient is not especially limited, and examples include a decongestant component, an eye muscle adjusting agent component, an anti-inflammatory agent component, an astringent component, an antihistamine component, an antiallergic agent component, vitamins, amino acids, an antimicrobial component, sugars, polymer compounds or derivatives thereof, cellulose or derivatives thereof, and a local anesthetic component.

The eyedrops can further contain one, two or more of various components and additives appropriately selected by a conventional method in accordance with the usage and form as long as the effects of the present invention are not impaired. Examples of these components and additives include various additives such as a carrier generally used in preparation of a liquid medicine, a perfume or cooling agent, a preservative, a bactericide or antibacterial agent, a pH adjuster, a chelating agent, a stabilizer, a tonicity agent, a buffer, and a thickening agent. Examples of representative components used in eyedrops include, but are not limited to, the following.

Examples of the carrier include water, and an aqueous solvent such as hydrous ethanol. When various components are difficult to be dissolved in an aqueous solvent, a solubilizing agent may be used. Examples of the solubilizing agent include polyoxyethylene hydrogenated castor oil, polyoxyl 40 stearate, povidone, and polysorbate 80.

Examples of the perfume or cooling agent include terpenes (specifically such as anethol, eugenol, camphor, geraniol, cineole, borneol, menthol, limonene, and borneo camphor; all of which may be in any of d-form, 1-form, and dl-form), and essential oils (such as fennel oil, cool mint oil, cinnamon oil, spearmint oil, peppermint water, mint oil, peppermint oil, bergamot oil, eucalyptus oil, and rose oil).

Examples of the preservative, and the bactericide or antibacterial agent include polidronium chloride, alkyldiaminoethylglycine hydrochloride, sodium benzoate, ethanol, benzalkonium chloride, benzethonium chloride, chlorhexidine gluconate, chlorobutanol, sorbic acid, potassium sorbate, sodium dehydroacetate, methyl parahydroxybenzoate, ethyl parahydroxybenzoate, propyl parahydroxybenzoate, butyl parahydroxybenzoate, oxyquinoline sulfate, phenethyl alcohol, benzyl alcohol, biguanide compounds (specifically such as polyhexamethylene biguanide, and hydrochlorides thereof), and Glow Kill (name of a product manufactured by Rhodia).

Examples of the pH adjuster include hydrochloric acid, sodium hydroxide, potassium hydroxide, calcium hydroxide, magnesium hydroxide, triethanolamine, monoethanolamine, diisopropanolamine, sulfuric acid, and phosphoric acid.

Examples of the chelating agent include ascorbic acid, tetrasodium edetate, sodium edetate, and citric acid.

Examples of the stabilizer include sodium edetate hydrate, povidone, polysorbate 80, dibutylhydroxytoluene, trometamol, sodium formaldehyde sulfoxylate (rongalite), tocopherol, sodium metabisulfite, monoethanolamine, aluminum monostearate, and glycerin monostearate.

Examples of the tonicity agent include potassium chloride, sodium chloride concentrated glycerin, glucose, and D-mannitol.

Examples of the buffer include sodium citrate hydrate, sodium acetate hydrate, sodium bicarbonate, trometamol, boric acid, borax, sodium hydrogen phosphate hydrate, and sodium dihydrogen phosphate.

Examples of the thickener include a carboxyvinyl polymer, povidone, polyvinyl alcohol (partially saponified), hydroxyethylcellulose, hypromellose, methylcellulose, and glycerin.

In the eyedrops of the present invention, these additives can be blended in expectation of the effects of the present invention, or as long as the effects of the present invention are not impaired. The content is not especially limited, and is preferably about 0.001 to 1% by mass based on the total amount of the eye drops.

The pH of the eyedrops may be 3 to 10, and is preferably 4 to 9 from the viewpoint of usability, and more preferably 5 to 8.5 from the viewpoint of usability.

As a container for holding the eyedrops of the present invention, any of known eyedroppers can be used without limitation. As an eyedropper, any container having a shape capable of dropping eyedrops onto an eye, for example, a shape having a nozzle and a container mouth at the tip of the nozzle can be used. Besides, the eyedropper may be either of one having a structure in which a separately molded nozzle is attached onto a container, and one having a structure in which a nozzle portion (liquid injection portion) and a container body are integrally molded (such as a disposable eyedropper).

The eyedropper may be usually a plastic container. The constituent material of the plastic container is not especially limited, and examples include one of polyethylene terephthalate, polyarylate, polyethylene naphthalate, polycarbonate, polyethylene, polypropylene, and polyimide, a copolymer of these, and a mixture of two or more of these. From the viewpoint that the effects of the present invention are easily exhibited depending on the extent of pushing out, polyethylene terephthalate, polyarylate, polyethylene naphthalate, a copolymer of these, or a mixture of two or more of these are particularly preferred.

The eyedrops may be filled in a transparent container (a container having transparency sufficient for observing a foreign matter therein) using such a material as a principal material, or may be filled in a light resistant container. The light resistance may be obtained, for example, by adding a colorant to a transparent container material, or may be obtained by covering the container with a shrink film, an outer case or the like. Besides, the volume of the container is preferably about 0.5 to 50 mL, and more preferably about 3 to 20 mL for more easily exhibiting the effects of the present invention depending on the extent of pushing out.

Besides, the nozzle provided on the eyedropper is also not especially limited in the structure and the constituent material. The structure of the nozzle may be any structure generally employed as the nozzle of an eyedropper. Besides, examples of the constituent material of the nozzle are similar to those described above regarding the constituent material of the plastic container. From the viewpoint of making more favorable the anti-drip property of the eyedrops, and suppressing variation in drop amount, a nozzle containing polyethylene or polypropylene as the constituent material is favorable. Examples of the type of polyethylene include high density polyethylene and low density polyethylene, and in particular, a nozzle containing low density polyethylene as the constituent material is favorable.

### (Method for Producing Eyedrops)

The eyedrops of the present invention can be prepared by a method commonly employed by or known to those skilled in the art. For example, the preparation may be performed by dispersing respective components in a carrier such as water, adding a solubilizing agent thereto if necessary, heating the resultant if necessary, homogenizing, dissolving or emulsifying the resultant with a homomixer or the like, and adjusting the pH with a pH adjuster. Besides, as a method for sterilizing the formulation, electron beam sterilization, autoclave sterilization, filtration sterilization or the like can be selected.

### (Usage)

The administration method and the dosage of the eyedrops of the present invention are varied depending on the symptom of a patient and the like, and about 1 to 2 drops each may be usually eye-dropped about once to 6 times a day.

Although not restricted, when the eyedrops of the present invention are used as eyedrops containing at least one selected from the group consisting of phenylbutyric acid and pharmacologically acceptable salts thereof as a therapeutic agent of scleral thinning, for example, 1 to 2 drops each can be eye-dropped once or twice a day, and it is preferable to eye-drop one drop each once a day.

Besides, from the viewpoint of remarkably exhibiting the effects of the present invention, the eyedrops of the present invention can be used, for example, in an inactive time period, for example, before a nap, before bedtime or the like.

### [Screening Method for Therapeutic Agent of Scleral Thinning]

In the present invention, a screening method for a therapeutic agent of scleral thinning includes a step of contacting a candidate substance with an eye-derived cell, and a step of selecting the candidate substance by using, as an indicator, change in a protein and/or a gene of a signal transduction system of PERK and/or ATF6 in the cell. Although not limited, for example, contact with the cell is caused in the presence of or in the absence of the candidate substance, and the change in the protein and/or the gene of the signal transduction system of PERK and/or ATF6 caused by the candidate substance is measured for comparison, and thus, the candidate substance can be screened.

The eye-derived cell is not limited, and from the viewpoint of remarkably exhibiting the effects of the present invention, is preferably a cell in the sclera.

Although not limited, the eye-derived cell is preferably a cell derived from an animal model where myopia has been induced. As such a myopia-induced model, a known animal model can be used.

Although not limited, examples of the myopia-induced model include an animal model caused to wear a minus lens to induce myopia, and an animal model in which myopia has been induced by administering a myopia inducing agent.

As such a minus lens, one having a power of -20 to -40 diopters (D) can be used, and the power is preferably -25 to -35 diopters (D). A method for causing the minus lens to be worn is not limited but any known methods can be employed, and for example, the minus lens is fixed in front of the eye of an animal with a fixing tool.

The period when the minus lens is worn is, for example, at least 1 week, preferably 2 weeks or more, and more preferably 3 weeks or more.

Besides, as the myopia inducing agent, any of known substances can be used, and for example, tunicamycin, thapsigargin, or the like can be used as the myopia inducing agent. Alternatively, as the myopia inducing agent, a PERK pathway activator and an ATF6 pathway activator can be used in combination. An example of the PERK pathway activator includes CCT020312, an example of the ATF6 pathway activator includes AA147, these can be administered singly or administered as a mixture, and it is preferable that these are administered as a mixture.

Although not limited, such a myopia inducing agent can be administered in the form of an injection or eyedrops from the viewpoint of causing it to act on an eye cell of the sclera or the like, and it is preferably administered in the form of eyedrops. When tunicamycin is used in the form of eyedrops, the concentration can be, for example, 10 to 100 µg/mL, and is preferably 20 to 80 µg/mL, and more preferably 40 to 60 µg/mL.

When thapsigargin is used in the form of eyedrops, the concentration can be, for example, 1 to 100 µM, and is preferably 2 to 60 µM, and more preferably 5 to 30 µM.

When a myopia-induction model is used, from the viewpoint that the animal model is used on the assumption of application to scleral thinning and an eye disease associated therewith, an animal in which myopia induction has been completed is preferably used. Although not limited, when a mouse is used, a period when a minus lens is started to be worn is preferably the weaning period, and the mouse is more preferably 3 weeks old. In a C57BL6 mouse or the like, the physiological axial elongation occurs from 3 weeks old to 6 weeks old. Therefore, when myopia is induced from 3 weeks old, excessive axial elongation can be accelerated in addition to the physiological axial elongation, and hence pathologic axial elongation can be thus caused.

Besides, the time of applying a candidate substance is, for example, preferably during the myopia induction (3 weeks old to 6 weeks old or the like), and/or after the myopia induction (6 weeks old to 8 weeks old or the like). From the viewpoint that the screening is performed in a period when a posterior segment eye disorder is caused by scleral thinning, which corresponds to adulthood in a human, the time of applying a candidate substance is more preferably, for example, after the myopia induction (6 weeks old to 8 weeks old or the like). Although not limited, when a method in which a candidate substance is applied after the myopia induction is employed, the influence of the candidate substance on the pathologic axial elongation remaining after completion of myopia progress in childhood, and on scleral thinning can be evaluated.

Although not limited, in the step of contacting a candidate substance with an eye-derived cell, the candidate substance is preferably administered orally, by intraperitoneal injection, or by eyedrops, and more preferably by eyedrops. For example, when evaluation is to be performed in a cell of the sclera, the candidate substance can be contained in eyedrops to be administered.

In a step of measuring change, caused by the candidate substance, in a protein, and/or a gene of the signal transduction system of PERK and/or ATF6, any known evaluation method can be employed. Although not limited, expression of gene, or expression or secretion of a protein can be measured by known methods such as microarray, real-time PCR method, PCR method, Western blotting method, ELISA method, and immunohistochemistry.

For example, when change in a gene of the signal transduction system of PERK or ATF6 is to be measured, RNA is extracted from a cultured cell by a known RNA extraction method to be supplied to a step of quantitatively analyzing expression of mRNA.

In the step of quantitatively analyzing expression of mRNA, real-time PCR method is preferably employed although not limited. As a marker to be measured by real-time PCR method, a factor related to signal transduction described above in (Inhibitor of PERK Pathway and ATF6 Pathway) can be used as a measurement item.

Examples of a factor related to the PERK pathway include CHOP, ATF4, and GADD34.

Examples of a factor related to the ATF6 pathway include GRP78, GRP94, PDI, Cnex, HYOU, and ERdj3.

In a step of measuring scleral thinning caused by the candidate substance, any known evaluation method can be employed. Although not limited, when the evaluation is performed based on the thickness of the sclera, a sclera tissue is stained by HE staining, the tissue is observed and analyzed with a microscope or the like, and thus, the thickness of the sclera can be measured. Alternatively, in order to measure the thickness of the sclera *in situ,* an optical coherence tomography apparatus (OCT) can be used, and for observing the thickness of the sclera more precisely, spectral domain (SD)-OCT or swept source (SS)-OCT can be used.

Although not limited, when the evaluation is performed based on the thickness or amount of collagen fibers in the sclera, the fiber area or the diameter in the fiber cross-section can be measured by observing and analyzing the collagen fibers in the sclera with an electron microscope or the like.

When scleral thinning is inhibited by the candidate substance, the candidate substance is selected as a therapeutic agent of scleral thinning, and can be used as a therapeutic agent of scleral thinning.

The present invention can be practiced also in the following aspects:
Eyedrops for treating scleral thinning, comprising an inhibitor of the PERK (PKR-like endoplasmic reticulum kinase) pathway and/or the ATF6 (activating transcription factor 6) pathway as an active ingredient;
eyedrops for use in treatment of scleral thinning, comprising an inhibitor of the PERK pathway and/or the ATF6 pathway as an active ingredient;
use of an inhibitor of the PERK pathway and/or the ATF6 pathway in production of eyedrops for treating scleral thinning;
a method for treating scleral thinning, comprising causing a human to take an effective amount of an inhibitor of the PERK pathway and/or the ATF6 pathway;
the eyedrops, the use, or the method described above, in which the inhibitor selectively inhibits at least the ATF6 pathway;
the eyedrops, the use, or the method described above, in which the inhibitor is an inhibitor of both the PERK pathway and the ATF6 pathway;
the eyedrops, the use, or the method described above, in which the inhibitor is at least one selected from the group consisting of phenylbutyric acid and pharmacologically acceptable salts thereof;
the eyedrops, the use, or the method described above, in which the inhibitor is sodium phenylbutyrate;
the eyedrops, the use, or the method described above, in which a content of the inhibitor is 0.01 to 5% by mass based on a total amount of the eyedrops;
the eyedrops, the use, or the method described above, in which a content of the inhibitor is 0.1 to 3% by mass based on a total amount of the eyedrops;
the eyedrops, the use, or the method described above, in which a content of the inhibitor is 0.2 to 2% by mass based on a total amount of the eyedrops;
the eyedrops, the use, or the method described above, in which the treatment of scleral thinning does not inhibit physiological axial elongation;
the eyedrops, the use, or the method described above, in which the treatment of scleral thinning inhibits pathologic axial elongation;
the eyedrops, the use, or the method described above, in which the treatment of scleral thinning is used for treatment of an eye disease associated with scleral thinning;
the eyedrops, the use, or the method described above, in which the eye disease associated with scleral thinning is myopic macular degeneration, myopic chorioretinal atrophy, myopic choroidal neovascularization, myopic optic neuropathy, myopic retinopathy, chorioretinal atrophy, macular hemorrhage, myopic traction maculopathy, myopic maculopathy, myopic macular lesion, myopic macular separation, myopic refractive scotoma, myopic conus, myopic foveoschisis, diffuse atrophic lesion, focal atrophic lesion, Lacquer cracks, posterior staphyloma, retinal detachment, macular hole, tilted disc syndrome, myopic astigmatism, fixed esotropia, mechanical abduction restriction, esotropia, or high myotic strabismus;
the eyedrops, the use, or the method described above, in which the eye disease associated with scleral thinning is myopic macular degeneration, myopic chorioretinal atrophy, myopic choroidal neovascularization, or myopic optic neuropathy;
the eyedrops, the use, or the method described above, in which the eyedrops are aqueous eyedrops;
the eyedrops, the use, or the method described above, in which the eyedrops are used to be eye-dropped once or twice a day;
the eyedrops, the use, or the method described above, in which the eyedrops are used to be eye-dropped once or twice a day;
the eyedrops, the use, or the method described above, in which the eyedrops are used before a nap or before bedtime;
a screening method for a therapeutic agent of scleral thinning, comprising a step of contacting a candidate substance with an eye-derived cell, and a step of selecting the candidate substance by using, as an indicator, change in a protein and/or a gene of the signal transduction system of PERK and/or ATF6 in the cell;
the screening method described above, in which the eye-derived cell is a cell derived from an animal model in which myopia has been induced;
the screening method described above, in which the animal model is an animal model in which myopia has been induced by causing a minus lens to be worn, or an animal model in which myopia has been induced by administering a myopia inducing agent;
the screening method described above, in which the minus lens is a lens having a power of -20 to -40 diopters (D);
the screening method described above, in which a period when the minus lens is worn is at least 1 week;
the screening method described above, in which the myopia inducing agent contains tunicamycin, and/or thapsigargin;
the screening method described above, in which a concentration of the tunicamycin administered by eyedrop is 10 to 100 µg/M;
the screening method described above, in which a concentration of the thapsigargin administered by eyedrop is 1 to 100 µM;
the screening method described above, in which the animal model starts to wear the minus lens in the weaning period;
the screening method described above, in which the step of contacting a candidate substance with an eye-derived cell includes administering the candidate substance orally, by intraperitoneal injection, or by eyedrop administration;
the screening method described above, comprising selecting the candidate substance as a therapeutic agent of scleral thinning when expression of the protein and/or the gene of the signal transduction system of PERK and/or ATF6 is inhibited by the candidate substance;
the screening method described above, comprising selecting the candidate substance as a therapeutic agent of scleral thinning when expression of the protein and/or the gene of the signal transduction system of PERK and ATF6 is inhibited by the candidate substance;
the screening method described above, comprising selecting the candidate substance as a therapeutic agent of scleral thinning when expression of collagen-related protein and/or gene in the sclera is normalized by the candidate substance; and
the screening method described above, in which the collagen-related protein and/or gene is at least one selected from the group consisting of COL1A1, COL4A3, COL8A2, COL11A2, and COL15A1.

### Examples

Now, the present invention will be specifically described by way of experimental results.

### [Experimental Method]

All animal experiments performed in the present experiment were approved by Institutional Animal Experiment Committee at KEIO University, and were performed in compliance with Guideline for Care and Use of Laboratory Animals of KEIO University, ARVO Statement for the Use of Animals in Ophthalmic and Vision Research, and Animal Research: Reporting of *in vivo* Experiments (ARRIVE) Guideline.

### (Features of Myopia-induced Mouse)

As described in Non Patent Literature 3, eyes of a human are hyperopic immediately after birth, and thereafter, the eye axes elongate (namely, become myopic), and the eyes become emmetropic in a school period (about 8 years old). Besides, also in a period of 3 to 6 weeks old of a mouse (C57BL6), the eye axes elongate in accordance with the growth, and this myopia induction period of 3 to 6 weeks old corresponds to childhood in a human in terms of movement of myopia progression, and a period after the myopia induction period substantially corresponds to adulthood. When such an animal model is used, the mechanism of the myopia progression (scleral thinning) in an adult human can be clarified, and a therapeutic agent of scleral thinning or a therapeutic agent of an eye disease associated therewith can be screened.

The mechanism for inducing axial myopia by causing a minus lens to be worn is schematically illustrated in Figure 1(a). Emmetropia refers to a state where an image is clearly seen because parallel rays entering the eyes is focused on the retina. On the other hand, axial myopia refers to a state where an image cannot be clearly seen because parallel rays entering the eyes are focused in front of the retina due to elongated eye axes. Eyes of animals, including a human, become large with growth. When a juvenile mouse is caused to wear a minus lens, the eye axis elongates to a focusing position obtained in wearing the minus lens, namely, up to a state where an image is clearly seen in wearing the minus lens. As a result, the eye axis elongates, and thus, an eye state similar to the state of an axial myopic eye can be created.

### (Production of Myopia-induced Mouse)

Specifically, a myopia-induced mouse is produced as follows. It is noted that myopia induction, and measurement of an axial length and refraction were performed by methods similar to those described in Non Patent Literatures 6 and 7. First, a male C57BL6J mouse was put in a standard transparent cage under a 12/12 h light/dark cycle in a temperature controlled clean room. The animal was allowed to free access to standard feed and autoclaved tap water. A 3-week-old mouse immediately after weaning was anesthetized with a mixed anesthetic of three anesthetics, that is, Domitor (Nippon Zenyaku Kogyo Co., Ltd.), Butorphanol (Meiji Seika Pharma Co., Ltd.), and Midazolam (Sandoz K.K.), and the skull was exposed with scissors. As illustrated in Figure 1(b), a post was provided to stand on the skull, and fixed with dental cements (Super-Bond, Sun Medical Company, Ltd.). The post was provided with a screw thread so that an adjustor described below could be fixed thereon with a nut.

For inducing myopia, a minus lens having a power of -30 diopters (D) (Rainbow Contact, RAINBOW OPTICAL LABORATORY CO., LTD.) was worn on the right eye (myopia-induced eye), and a lens having a power of 0 D or only a frame was worn as a control on the left eye (control eye). A protector in a shape projecting sideways was attached to a frame portion below the lens so that the mouse did not damage the lens with the forelegs or the like when the lens was worn on the mouse. Owing to the protector, the mouse could not touch the lens, and hence the lens was not damaged. The protector used here was attached to be integral with the frame portion, but the protector needs not be integral with the lens as long as the lens is not damaged by the behavior of the mouse. For example, the protector may be in a shape similar to an Elizabeth collar worn by an injured animal.

In the frame portion above the lens, the adjustor for adjusting the width and the angle of the worn lens in accordance with the growth of the mouse was attached. The adjuster was in a bent dogleg shape, one end thereof was attached to the lens, and the other end thereof was provided with a slotted hole so that it could be attached to the post provided to stand on the head. When the post was inserted through the slotted hole to be screwed with a nut, the adjustor could be fixed to be close contact with the skin without pressing the peripheries of the eye of the mouse. Owing to the adjustment mechanism composed of the post, the nut and the adjustor, the width and the angle could be adjusted in accordance with the growth of the mouse, so that the lens could be disposed in a position corresponding to the eye of the mouse. Besides, since the lens was removable, change over time in the axial length and the refraction value could be measured.

### (Measurement of Axial Elongation and Refraction)

After causing the frame alone to be worn on the control eye, and a 30 D lens to be worn on the myopia-induced eye for 3 weeks, the refraction value and the axial length were measured to obtain differences caused through the wear. The refraction value was measured with a refractometer (Infrared Photorefractor for Mice, produced by Professor Schaeffel, Tubingen University), and the axial length was measured with SD-OCT (Spectral-domain OCT, spectral-domain optical coherence tomography, Envisu R4310, Bioptigen Inc.).

### (Preparation of Sclera Sample)

After experimental intervention and eye parameter measurement, the eyeball was excised from the C57BL6J mouse. For transmission electron microscopic observation, the eyeball of the mouse was fixed in ice-cooled 2.5% glutaraldehyde in PBS for 1 hour, and then was cut along the sagittal plane. The cornea and the lens were excised, and the remaining tissue was further fixed in 2.5% glutaraldehyde/60 mM HEPES buffer (pH 7.4) overnight. The resultant specimen was washed with 60 mM HEPES buffer, and was then incubated in 1% tetroxide osmium/60 mM HEPES buffer at 4°C for 2 hours, and the resultant was dehydrated through ethanol series, exchanged and embedded in Epon 812 (EM Japan, Tokyo, Japan). After the embedding, the resultant block was sliced into 70 nm, and stained with uranyl acetate and lead citrate. The resultant section was visualized with JEM-1400Plus (JEOL LTD., Tokyo, Japan).

In order to prepare a frozen section (mouse), the eyeball was frozen with an OCT compound (Sakura Finetek, Tokyo, Japan). The frozen block was cut into a thickness of 5 µm with a cryostat (CM3050S; Leica Biosystems, Wetzlar, Germas). The resultant section was stored at -80°C until use.

For producing a paraffin section (mouse), the eyeball was fixed in 4% paraformaldehyde overnight, the resultant was embedded in paraffin, and sliced into a 3 µm section with a microtome (REM-710, Yamato Kohki, Saitama, Japan). Next, the section was stained with hematoxylin and eosin, and visualized with BX53 microscope (Olympus, Tokyo, Japan). The thickness of the sclera was measured using cellSens software (Olympus).

### (Preparation of Test Drug)

Sodium phenylbutyrate (Cayman Chemical, MI, USA) and tauroursodeoxycholic acid (Sigma Aldrich, Tokyo, Japan) were dissolved in PBS. STF080310 (Selleck Biotech, Tokyo, Japan) or 4µ8C (Selleck Biotech) of an IRE1 inhibitor, GSK2656157 (Selleck Biotech) or GSK2606414 (Selleck Biotech) of a PERK inhibitor, and Nelfinavir Mesylate hydrate (Tokyo Chemical Industry Co., Ltd.) or Ceapin-A7 (Sigma Aldrich) of an ATF6 inhibitor were dissolved in DMSO, and the resultant was diluted to 1: 1000 with PBS to be used in an eyedrop test.

### (Eyedrop Administration of URP Inhibitor during Myopia Induction)

During the myopia induction, 60 µM STF080312 (STF), 100 µM 4µ8C (4µ8C), 100 µM GSK2656157 (GSK), 100 µM GSK2606414 (GSK2606414), 100 µM Nelfinavir Mesylate hydrate (NFV), or 100 µM Ceapin-A7 (Ceapin) was eye-dropped to both eyes once a day every day in the evening for 10 days.

### (Intraperitoneal Injection)

A solution of sodium phenylbutyrate in PBS (4-PBA; 200 mg/kg) or tauroursodeoxycholic acid (TUDCA; 100 mg/kg) was intraperitoneally injected (i.p.) every day during the myopia induction period.

### (Eyedrop Administration of Sodium Phenylbutyrate after Myopia Induction Period)

A solution of 2% sodium phenylbutyrate in PBS (4-PBA) was eye-dropped to both eyes of a myopia induced mouse once a day every day in the evening for 10 days after completing the myopia induction period corresponding to human adulthood. It is noted that administration schedule during the myopia induction period (LIM) and after completing the period is schematically illustrated in Figure 11.

### (Concentration Measurement and Analysis by Western Blotting Method)

A protein (10 µg/well) of the sclera was separated by SDS-PAGE, transferred onto a PVDF membrane (Merck Millipore, MA, USA), blocked with Blocking One (Nacalai Tesque, Tokyo), and incubated together with anti-ATF6 (BioAcademia), phosphorylation-IRE1 (Ser724, Abeam, Cambridge, UL), IRE1, phosphorylation-eIF2a, eIF2a, and β-actin (Cell Signaling Technologies Japan, Tokyo, Japan) antibodies at 4°C overnight. The resultant membrane was incubated with an appropriate HRP-bound secondary antibody, and the resultant was visualized with EzWestLumi plus (ATTA, Tokyo, Japan). SDS-PAGE was performed in a 10% acrylamide gel with a protein size marker (Magic Mark XP Western Protein Standard, ThermoFisher Scientific). The concentration measurement and analysis was performed using Image J software.

### (Quantitative PCR)

Quantitative real-time PCR was performed using StepOnePlus Realtime PCR system with PowerUp SYBR Green Master Mix (Applied Biosystems, CA, USA). Expression level was standardized using β-actin.

### (Statistical Analysis)

Data obtained in the experiment were all indicated as an average ± standard deviation. A difference between groups was analyzed by Student's t-test, one-way analysis of variance, or using a generalized estimating equation. When a significant difference was obtained by ANOVA, Tukey HSD was next performed to determine significance of a difference between averages. A p value of less than 0.05 indicates a statistically significant difference.

### [Experimental Results]

### <Test Example 1 Scleral Thinning after Myopia Induction in Myopia-induced Mouse>

It is said that thinning of the sclera that maintains the shape of the eyeball is an early symptom of deformation of the eyeball in high myopia (see Non Patent Literature 4). Therefore, in order to confirm that the thinning had been caused also in the sclera of a myopia-induced mouse, the sclera after the myopia induction was histologically evaluated.

In accordance with the test method described above, the axial elongation and the refraction change after myopia induction in a myopia-induced mouse were evaluated (Figure 2). Besides, the eyeball after the myopia induction was excised, and stained with hematoxylin and eosin to observe the thickness of the sclera (Figures 3a and 3b). In addition, the thicknesses of the sclera at respective distances from the nerve papilla (disk) were plotted in a graph (Figure 3c).

As a result, the sclera was significantly thinned over substantially the whole periphery in the myopia-induced eye as compared with the control eye (Figure 3c). As shown in these results, it was confirmed that when the eye axis pathologically elongates due to myopia, the sclera is thinned, which causes an early symptom of the deformation of the eyeball that can cause various posterior segment eye diseases.

### <Test Example 2 Axial Elongation by Inhibition of URP Gene in Myopia-induced Mouse>

It is said that increase of expression of URP gene pathway is a cause of pathologic axial elongation and scleral thinning in high myopia (Patent Literature 1). Therefore, in this Test Example 2, it was evaluated, in the sclera of a myopia-induced mouse in which scleral thinning had been caused, how the phenotype of axial elongation was affected by a known URP gene inhibitor. It is noted that GSK2656157 (GSK) and GSK2606414 were used as the PERK inhibitor, Nelfinavir Mesylate hydrate (NFV) and Ceapin-A7 were used as the ATF6 inhibitor, and STF080312 (STF) and 4µ8C were used as the IRE1 inhibitor (Figure 4).

In accordance with the test method described above, the axial elongation caused in using each of the various gene inhibitors in a myopia-induced mouse was evaluated (Figure 4).

Figures 5a and 5b illustrate axial elongation (a) and refraction change (b) obtained after eyedrop administration of 60 µM STF, 100 µM GSK, or 100 µM NFV once a day for 10 days during the myopia induction period of the mouse. Besides, Figures 5c and 5d illustrate axial elongation (a) and refraction change (b) obtained after similarly eyedrop administration of a combination of these inhibitors (STF + GSK: S + G, GSK + NFV: G + N, NFV + STF: N + S, or STF + GSK + NTF: S + G + N). Besides, Figure 6 illustrates axial elongation (a) and refraction change (b) obtained after similarly eyedrop administration of 100 µM GSK2606414, Ceapin-A7, or 4µ8C.

As a result, when each of the inhibitors of UPR genes of PERK, ATF6 and IRE1 was singly eye-dropped, neither inhibition of axial elongation nor inhibition of myopic refraction was found in the sclera of the myopia-induced mouse contrary to expectation (Figures 5a and 5b). Instead, when each of the inhibitors excluding STF was singly eye-dropped, the eye axis of the control eye in which myopia had not been induced significantly elongated to cause myopic refraction as compared with that caused by eyedrop administration of DMSO. Besides, when a combination of these was eye-dropped, only when at least two of the PERK inhibitors and the ATF6 inhibitors (G + N, and S + G + N) were included in the combination, axial elongation was significantly inhibited, myopic refraction was inhibited, and the axial elongation caused in the control eye, which was caused by eyedrop administration of a single one of these, was not caused (Figures 5c and 5d). In addition, axial elongation and refraction change caused by eyedrop administration of single one of the inhibitors GSK2606414, Ceapin-A7, and 4µ8C, which are different from those used in Figure 5, were completely the same as the results illustrated in Figure 5, and neither axial elongation nor myopic refraction was inhibited, and the eye axis of the control eye also elongated when each of those excluding 4µ8C was singly eye-dropped (Figure 6).

It was confirmed, based on these results, that axial elongation is inhibited and myopic refraction is inhibited when at least both PERK and ATF6, among the URP genes, were inhibited. It was confirmed that pathologic axial elongation is caused by inhibiting PERK singly, ATF6 singly, a combination of PERK and IRE1, and a combination of ATF6 and IRE1. This is probably because inhibition of both the PERK pathway and the ATF6 pathway is significant for the inhibition of axial elongation but when one of these is inhibited, the other is overactivated in compensation, and hence axial elongation cannot be inhibited in total, and hence, it was regarded that inhibition of both the PERK pathway and the ATF6 pathway is essential for the inhibition of scleral thinning. Furthermore, it was newly found that it is necessary to search a component capable of inhibiting at least both the PERK pathway and the ATF6 pathway for searching a therapeutic agent of scleral thinning. Besides, since the same results were obtained by using the different inhibitors, it is regarded that the simultaneous inhibition of the PERK pathway and the ATF6 pathway being effective for inhibition of scleral thinning is not limited to specific inhibitors but is a universal mechanism.

### <Test Example 3 URP Gene Expression Inhibition Effect of 4-PBA in Sclera of Myopia-induced Mouse>

It was confirmed in Test Example 2 that it is necessary to search a component inhibiting at least both the PERK pathway and the ATF6 pathway for searching a therapeutic agent of scleral thinning, and therefore, in this Test Example 3, components conventionally known to inhibit axial elongation (sodium phenylbutyrate {4-PBA}, and tauroursodeoxycholic acid {TUDCA}) were evaluated for inhibition profile against the UPR genes.

Figure 7 illustrates change in gene expression obtained after intraperitoneal injection of 4-PBA every day through the myopia induction period. Figure 8 illustrates axial elongation (a) and refraction change (b) similarly caused by 4-PBA administration in 1st week and 3rd week of the myopia induction. Figure 9 illustrates axial elongation (a) and refraction change (b) similarly caused by TUDCA administration in 1st week and 3rd week of the myopia induction.

As a result, the expression of genes disposed downstream of the PERK pathway and the ATF6 pathway overactivated in the myopia-induced mouse (Figure 4) was significantly inhibited by eyedrop administration of 2% 4-PBA (Figure 7). Besides, the axial elongation caused by eyedrop administration of 2% 4-PBA in 1st and 3rd weeks was significantly inhibited to the same extent, and myopic refraction was also significantly inhibited to the same extent. Besides, axial elongation of the control eye in which myopia had not been simultaneously induced (physiological axial elongation) was not inhibited but pathologic axial elongation of the myopia-induced eye alone was inhibited (Figure 8). Similarly, physiological axial elongation was not inhibited but pathologic axial elongation alone was inhibited by administration of TUDCA, which is known as a UPR gene inhibitor, similarly to 4-PBA (Figure 9).

It was confirmed, based on these results, that 4-PBA and TUDCA inhibit only pathologic axial elongation by inhibiting the PERK pathway and the ATF6 pathway corresponding to the mechanism of scleral thinning in the URP gene pathways. In other words, it was suggested that scleral thinning and a posterior segment eye disease associated therewith can be treated by a component inhibiting at least both PERK and ATF6 such as a combination of 4-PBA, TUDCA, a PERK inhibitor and an ATF6 inhibitor.

### <Test Example 4 Effect of Inhibiting Collagen Fiber Narrowing by 4-PBA in Sclera of Myopia-induced Mouse>

The sclera is a tissue mainly containing collagen, and it is said that collagen fiber narrowing is involved in scleral thinning (see Non Patent Literature 5). Therefore, in this Test Example 4, when PBS was intraperitoneally injected into a mouse every day over the myopia-induced period, collagen fibers were narrowed in the sclera of the myopia-induced eye, but when 4-PBA was similarly administered, the narrowing of collagen fibers in the sclera was inhibited, and the collagen fibers restored to the original thickness (Figure 10a). When the cross-sectional area of each thickness of these collagen fibers was calculated, the total area of thin collagen fibers having a cross-sectional area of 8000 µm² or less was increased by causing myopia (many fibers were thin with -30 D of PBS), but the area profile was restored to the original one by eyedrop administration of 2% 4-PBA (Figure 10b).

It was confirmed, based on these results, that the sclera is thinned when the eye axis is pathologically elongated by myopia. Besides, it was confirmed that the administration of 4-PBA has an effect of cancelling the scleral thinning.

### <Test Example 5 Effect of Inhibiting Pathologic Axial Elongation of 4-PBA after Myopia Induction in Sclera of Myopia-induced Mouse>

It was evaluated whether or not axial elongation is inhibited by eyedrop administration of 4-PBA after the myopia induction period (6 weeks old to 8 weeks old), namely, in a period, corresponding to adulthood in a human, when a posterior segment eye disorder is caused by scleral thinning (Figure 11) differently from the eyedrop administration in the myopia induction period (3 weeks old to 6 weeks old) in a mouse model simulating myopia progression in childhood as described in Patent Literature 1.

After completing myopia induction, 2% 4-PBA was eye-dropped every day, and axial elongation inhibition in 1st and 3rd weeks is illustrated in Figure 12a, and refraction change is illustrated in Figure 12b.

As a result, it was found that the eye axis continuously elongated even 3 weeks after completing the myopia induction, and that eyedrop administration of 2% 4-PBA tended to inhibit what is called adulthood axial elongation (Figure 12a). Besides, myopic refraction was also caused after completing the myopia induction, and similarly, tended to be inhibited by eyedrop administration of 2% 4-PBA (Figure 12b).

It was suggested, based on these results, that the progression of myopia remains even in adulthood after completing the myopia progression in childhood, and that 2% 4-PBA can inhibit this excessive axial elongation, and can treat scleral thinning and a posterior segment eye disease associated therewith.

Based on the results of Test Examples 1 to 5, it was confirmed that scleral thinning causing deformation of the eyeball is caused in the mouse myopia induction model, and that it is caused due to narrowing of collagen fibers in the sclera. It was considered that URP gene group is involved in these mechanisms, and that it is significant to simultaneously inhibit the PERK pathway and the ATF6 pathway among the UPR genes for inhibiting scleral thinning. These results reveal that the mouse myopia induction model is a test system useful for searching a therapeutic agent of scleral thinning and a posterior segment eye disease associated therewith.

Besides, it was confirmed that pathologic axial elongation causing scleral thinning continues even in a period corresponding to adulthood after childhood myopia progression, and this is considered as the mechanism of scleral thinning and a posterior segment eye disease associated therewith. It was confirmed that 4-PBA inhibits not only the myopia progression in childhood but also pathologic axial elongation in adulthood, and in addition, has an effect of restoring narrowing of collagen fibers caused by the myopia induction. These results reveal that 4-PBA is useful as a therapeutic agent of scleral thinning and a posterior segment eye disease associated therewith.

### <Test Example 6 Pharmacokinetics of 4-PBA>

Pharmacokinetics in the eye tissue obtained by systemic administration of 4-PBA was confirmed. A specific test method was as follows.

4-PBA (200 mg/kg) was intraperitoneally administered for 1 week. One hour after the last administration, the eyeball was excised, and respective tissues were separated. Tissues from 16 mice (32 eyeballs) were pooled to be used as 1 sample, and frozen with liquid nitrogen to be stored until measurement. Before the measurement by LC-MS/MS, a 9-fold weight of methanol/water (1:1, v/v) was added thereto for homogenization, and the resultant was centrifuged (10000 xg, 4°C, 5 minutes) to use a supernatant in analysis.

As a standard reagent, 4-phenylbutyric acid (Tokyo Chemical Industry Co., Ltd.) was used, and LC-MS/MS (LC-20AD system: Shimadzu Corporation, API4000: SCIEX, Tokyo, Japan), and HPLC column, Atlantis dc18 (5 µm, 2.1 mm ID x 150 mm, Waters) were used. As a mobile phase, formic acid/water (1:10000, v/v) and acetonitrile were used, and during the analysis, the gradient of each mobile phase was maintained at 50%. A sample injection amount was set to 10 µL, a column temperature was set to 50°C, and a flow rate was set to 0.2 mL/min.

As mass spectrometry (MS) conditions, negative mode electrospray ionization (ESI) was employed, and ion was measured by multiple reaction monitoring (MRM). The results are shown in Table 1.

### [Table 1]

**Table 1**

| | Sample | Time after Administration | 4-PBA Concentration (ng/g or ng/mL) | | | |
|---|---|---|---|---|---|---|
| | | | Retina (right) | Choroid | Sclera | Plasma |
| 4-PBA | 1 | - | undetectable | undetectable | undetectable | undetectable |
| | 2 | 15 min | 349 | 348 | 203 | 394 |
| | 3 | 30 min | 88.2 | 117 | 107 | 186 |
| | 4 | 60 min | 27.9 | 76.7 | 44.9 | 76.2 |

As shown in Table 1, when 4-PBA was intraperitoneally administered, 4-PBA was detected not only in the retina and the choroid but also in the sclera.

### <Test Example 7-1 Influence in vivo on Principal Collagen Components>

4-PBA was administered by eyedrop to a mouse myopia induction model to confirm its influence on collagen 1A1 (COL1A1) and the like corresponding to principal collagens for maintaining the shape of the eyeball. A specific test method was as follows.

PBS (Veh) or 2% 4-PBA was eye-dropped to a myopia-induced mouse, and collagen 1A1 in the sclera (n = 7) was evaluated by Western blotting method, and expression of collagen-related mRNA was evaluated by quantitative PCR.

The results are illustrated in Figure 13. As illustrated in Figure 13(A) and Figure 13(B), collagen 1A1 (COL1A1) protein and mRNA were reduced in the sclera of the myopia-induced eye. On the other hand, it was found that the reduction of collagen 1A1 protein and mRNA induced by myopia can be cancelled by administering 4-PBA by eyedrop. When similar evaluation was performed also on mRNAs of Col4a3, Col8a2, Co111a2, and Co115a1, the expression of these genes were increased by the myopia induction, and the increase was cancelled by the administration of 4-PBA by eyedrop (Figure 13(C)). These results show that the administration of 4-PBA by eyedrop inhibits scleral thinning by normalizing abnormal expression of the collagen protein group caused by myopia, and 4-PBA can be a therapeutic agent/preventive agent for a posterior segment eye disease associated therewith.

### <Test Example 7-2 Influence in vitro on Principal Collagen Components>

To human primary sclera fibroblast having been treated with tunicamycin capable of activating the same UPR pathway as myopia induction, an inhibitor specific to each of the UPR pathways was administered to confirm influence on collagen 1A1 (COL1A1) and the like whose expression was reduced by myopia induction in Test Example 7-1. A specific test method was as follows.

Human primary sclera fibroblast (huScF) (Lifeline Cell Technology, USA) was grown in FibroLife S2 fibroblast medium (Lifeline Cell Technology). The resultant cell was treated with 200 ng/mL of tunicamycin for 6 hours, and to the resultant, DMSO, or STF (IER1 inhibitor), GSK (PERK inhibitor), or NFV (ATF6 inhibitor) as a UPR gene inhibitor was administered, and proteins were collected from the resultant to perform Western blotting.

The results are illustrated in Figure 13. As illustrated in Figure 13(D) and Figure 13(E), collagen 1A1 (COL1A1) protein and mRNA were reduced by the tunicamycin treatment. Besides, the expression of mRNAs of Col4a3, Col8a2, Col11a2, and Col15a1 was increased by the tunicamycin treatment. These results accord with the expression profiles in the sclera tissue of the myopia induced eyes obtained in Test Example 7-2. Besides, when two components of GSK and NFV were administered (to inhibit PERK and ATF6), the reduction of the collagen 1A1 protein having been induced by tunicamycin was cancelled, and the expression of the collagen was found to be strongly increased. This result accords with a result of remarkable scleral thinning inhibition obtained when two components of GSK + NFV were administered to a myopia-induced mouse in Test Example 8 described below, and shows that the simultaneous inhibition of the PERK pathway and the ATF6 pathway is extremely effective for inhibiting scleral thinning by normalization of collagen secretion.

### <Test Example 8 Involvement of ATF6 Pathway in Treatment of Scleral Thinning and Posterior Segment Eye Disease Associated Therewith>

It was further examined how the ATF6 pathway, out of the pathways of the PERK pathway and the ATF6 pathway, is involved in treatment of scleral thinning and a posterior segment eye disease associated therewith. In accordance with the description given above in [Experimental Method], the concentration measurement and analysis was performed by Western blotting method to obtain the amount of activated form of ATF6 (ATF6-N) and the amount of precursor form ATF6 (ATF6-P). The method by eyedrop administration of single one of PERK, ATF6, and IRE1 inhibitors or the method by eyedrop of a combination of these inhibitors in the sclera of a myopia-induced juvenile mouse was the same as those described in Test Example 2.

A value obtained by dividing the amount of activated form of ATF6 (ATF6-N) by the amount of precursor form of ATF6 (ATF6-P) is illustrated as an activation indicator in Figure 14.

As illustrated in Figure 14, the value obtained by dividing the amount ATF6-N of activated form of ATF6 by the amount ATF6-P of precursor form of ATF6 was significantly large in some groups. The groups having the large values accord with the groups illustrated in Figure 5c and Figure 5d in which pathologic axial elongation and refraction value reduction were respectively caused. In other words, it is revealed that when the eye in which myopia has not been induced (non-LIM eye) has myopia (axial elongation or refraction value reduction), ATF6 is always activated (ATF6-N being larger than ATF6-P), and that when ATF6 is inactivated in a myopia-induced eye (LIM eye), myopia is inhibited. This correlation is found through comparison between Figures 5c and 5d and Figure 14, and is summarized as shown in Table 2 below. In Table 2, "STF" or "S" indicates the IRE1 inhibitor, "GSK" or "G" indicates the PERK inhibitor, and "NFV" or "N" indicates the ATF6 inhibitor in the same manner as in Test Examples described above. The results summarized in Table 2 show that scleral thinning associated with axial elongation is triggered by activation of the ATF6 pathway in the sclera of a myopic eye, and on the contrary, that scleral thinning associated with axial elongation is inhibited by inactivation of the ATF6 pathway. For treatment of a posterior segment eye disease associated with scleral thinning, a drug having such pharmacological effects (4-PBA) is effective.

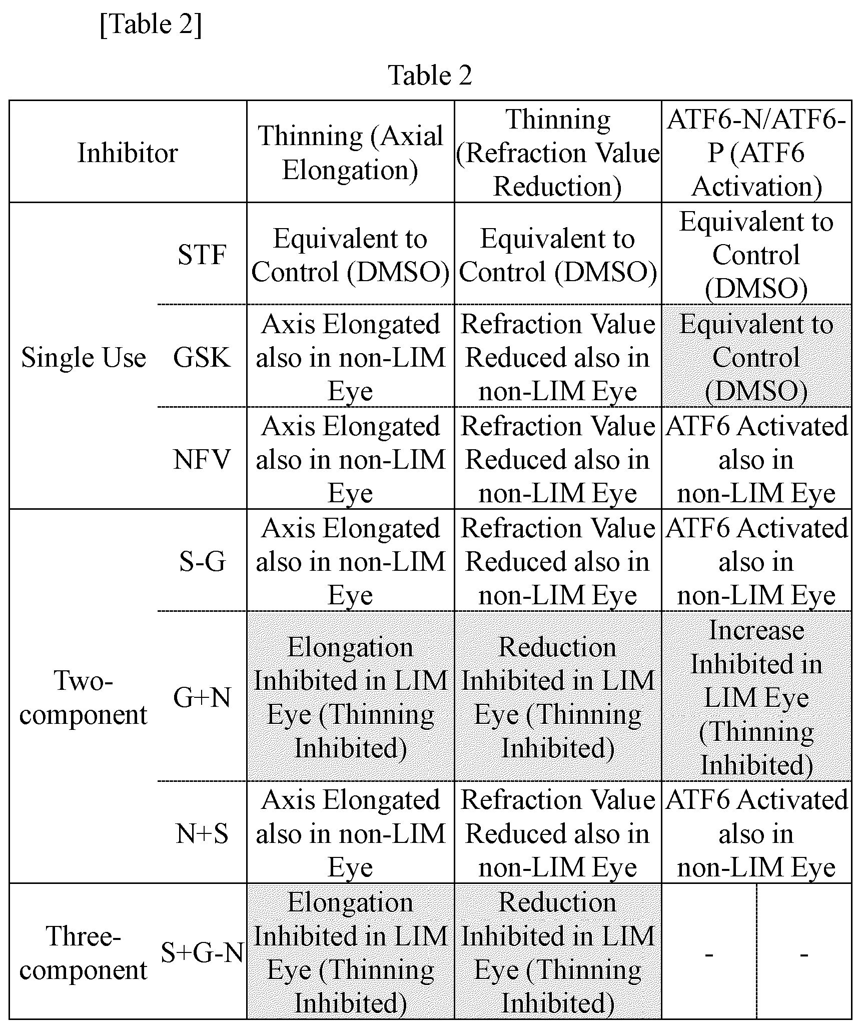

### <Test Example 9 Influence of Eyedrop Administration on Lens Thickening Caused by Myopia Induction>

It has been confirmed that the lens tends to be slightly thickened by myopia induction (LIM). It was examined whether or not a difference in the dosage form of 4-PBA affects the change in the lens. In accordance with the description given above in "Experimental Method", 4-PBA was eye-dropped or intraperitoneally administered to a myopia-induced juvenile mouse, and the thickness of the lens was measured with SD-OCT in the same manner as in the measurement of the axial length.

As illustrated in Figure 15(A), it was confirmed that the lens is thickened through myopia induction (LIM) in -30 D lens wearing group as compared with that in DMSOP eye-dropped NL (= no lens) group. Intraperitoneal administration of 4-PBA did not affect lens thickening. On the other hand, as illustrated in Figure 15(B), when 4-PBA was administered by eyedrop, lens thickening was not caused in the -30 D lens wearing group. In other words, it was revealed that eyedrop administration is favorable as the method for administering 4-PBA from the viewpoint of reachability to a target tissue.

## Claims

1. Eyedrops for treating scleral thinning, comprising an inhibitor of PERK (PKR-like endoplasmic reticulum kinase) pathway and/or ATF6 (activating transcription factor 6) pathway as an active ingredient.

2. The eyedrops according to claim 1, wherein the inhibitor is at least one selected from the group consisting of phenylbutyric acid and pharmacologically acceptable salts thereof.

3. The eyedrops according to claim 1 or 2, wherein the inhibitor is sodium phenylbutyrate.

4. The eyedrops according to any one of claims 1 to 3, wherein a content of the inhibitor is 0.01 to 5% by mass based on a total amount of the eyedrops.

5. The eyedrops according to any one of claims 1 to 4, wherein the treatment of scleral thinning is treatment of a posterior segment eye disease caused by the scleral thinning.

6. The eyedrops according to claim 5, wherein the posterior segment eye disease is myopic macular degeneration, myopic chorioretinal atrophy, myopic choroidal neovascularization, or myopic optic neuropathy.

7. A screening method for a therapeutic agent of scleral thinning, comprising a step of contacting a candidate substance with an eye-derived cell; and a step of selecting the candidate substance by using, as an indicator, change in a protein and/or a gene of a signal transduction system of PERK and/or ATF6 in the cell.
